(19) **Europäisches Patentamt**
**European Patent Office**
**Office européen des brevets**

(11) **EP 1 595 553 B1**

(12) # FASCICULE DE BREVET EUROPEEN

(45) Date de publication et mention
de la délivrance du brevet:
**25.04.2007 Bulletin 2007/17**

(51) Int Cl.:
*A61L 24/00* *(2006.01)*    *A61L 27/44* *(2006.01)*

(21) Numéro de dépôt: **05352009.4**

(22) Date de dépôt: **10.05.2005**

(54) **Ciment polmère pour la vertébroplastie percutanée**

Polymerer Zement für die perkutane Vertebroplastie

Polymeric cement for percutaneous vertebroplasty

(84) Etats contractants désignés:
**AT BE BG CH CY CZ DE DK EE ES FI FR GB GR HU IE IS IT LI LT LU MC NL PL PT RO SE SI SK TR**

(30) Priorité: **14.05.2004 FR 0405249**

(43) Date de publication de la demande:
**16.11.2005 Bulletin 2005/46**

(73) Titulaire: **Ceravic**
**65500 Vic en Bigorre (FR)**

(72) Inventeurs:
• **Leonard, Alain**
**6500 Caixon (FR)**
• **Lavergne, Claudine**
**65500 Caixon (FR)**

(74) Mandataire: **Morelle, Guy Georges Alain**
**Cabinet Morelle & Bardou, SC,**
**BP 72253**
**31522 Ramonville Cédex (FR)**

(56) Documents cités:
• **JASPER L E ET AL.: "Material properties of various cements for use with vertebroplasty" JOURNAL OF MATERIALS SCIENCE. MATERIALS IN MEDICINE, vol. 13, no. 1, janvier 2002 (2002-01), pages 1-5, XP002312782**
• **GARCIA CARRODEGUAS R ET AL.: "Injectable acrylic bone cements for vertebroplasty with improved properties" JOURNAL OF BIOMEDICAL MATERIALS RESEARCH, vol. 68, no. 1, 15 janvier 2004 (2004-01-15), pages 94-104, XP002312783**
• **MATHIS J.M. ET AL.: "Percutaneous Vertebroplasty: A Developing Standard of Care for Vertebral Compression Fractures" AMERICAN JOURNAL OF NEURORADIOLOGY, vol. 22, février 2001 (2001-02), pages 373-381, XP002312784**

EP 1 595 553 B1

## Description

La présente invention se rapporte au domaine des ciments polymériques, en particulier des ciments acryliques, utilisés pour la réparation des traumatismes des os et des articulations.

Elle a pour objet un ciment fluide à usage médical pour la reconstruction osseuse, notamment pour le comblement du corps vertébral, ainsi qu'une composition binaire destinée à la préparation d'un tel ciment. Un autre objet de la présente invention est un dispositif de conditionnement de ladite composition binaire. Un procédé de préparation d'un ciment osseux à partir d'une composition binaire est également revendiqué.

Les ciments osseux sont utilisés depuis de nombreuses années pour favoriser la fixation des implants artificiels au squelette. Le ciment servant de jonction entre l'os et l'implant doit répondre à un certain nombre d'exigences, notamment mécaniques, mais aussi être non toxique et biocompatible. Certains ciments ont même été étudiés pour leurs propriétés bioactives, c'est-à-dire pour leur action favorisant l'adhésion et la croissance cellulaire sur l'implant.

Depuis le milieu des années 80, l'emploi des ciments s'est étendu à la réparation osseuse, et tout en premier lieu à la vertébroplastie percutanée. Cette technique peu invasive, permet d'injecter un ciment à travers un trocart dans une vertèbre fracturée, pour assurer un volume osseux et une stabilisation. La première vertébroplastie percutanée a été réalisée en 1984 et a connu depuis un succès croissant, ouvrant la voie à la réparation plastique d'autres types d'os.

Les ciments utilisés jusqu'à présent sont des polymères organiques, formés à partir d'un mélange d'un prépolymère, généralement du PMMA (polyméthylméthacrylate de méthyle) et d'un monomère, généralement du MMA (méthylméthacrylate de méthyle), réagissant en présence d'un activateur de polymérisation. Pour une utilisation *in vivo* interdisant les températures élevées, un initiateur de réaction est ajouté.

La plupart des ciments disponibles dans le commerce sont présentés sous forme de deux composants séparés: une poudre comprenant principalement des billes de prépolymère, et un liquide contenant principalement le monomère. On incorpore en général l'initiateur, par exemple du péroxyde de benzoyle (BPO) à la poudre, tandis que le liquide contient un activateur chimique (catalyseur) tel que la diméthyl-para-toluidine (DMPT), la réaction de polymérisation démarrant lorsque les deux composants sont mélangés. Pour éviter une polymérisation spontanée pouvant se produire éventuellement durant le stockage, on incorpore en outre au composant liquide un stabilisant, communément l'hydroquinone. L'activateur et l'initiateur sont introduits à raison de 1 à 2 % dans le composant correspondant, le stabilisant faisant quant à lui effet à quelques dizaines de ppm.

Afin de visualiser le ciment durant et après l'opération par des moyens radiologiques, une substance radio-opaque peut être ajoutée, le plus souvent du sulfate de barium ($BaSO_4$) ou du dioxyde de zirconium ($ZrO_2$). Les ciments du commerce en contiennent une quantité de l'ordre de 10% dans la poudre.

Ces compositions binaires pour la préparation de ciments osseux, conçues à l'origine pour la fixation d'implants et le scellement de prothèse, répondent à des critères de résistance à la traction et à la compression, de neutralité chimique et de biocompatibilité. Elles sont homologuées pour un usage médical et ont prouvé leurs qualités à long terme lorsque le squelette est soumis à des efforts importants et répétés. C'est pourquoi les ciments osseux pour la fixation d'implant ont été adoptés comme matériau de prédilection pour la chirurgie de reconstruction des os.

Cependant, les conditions de mise en oeuvre des ciments en chirurgie percutanée impliquent que soient respectées un certain nombre d'exigences spécifiques, sous peine d'accidents dont les effets peuvent être dramatiques pour le patient, comme les paraplégies. C'est pourquoi le praticien doit disposer d'un ciment suffisamment fluide pour qu'il s'écoule à travers un trocart de quelques millimètres de diamètre, et qui conserve cette fluidité assez longtemps pour que le praticien ait le temps d'opérer en toute quiétude. Ce faisant, les caractéristiques physiques du ciment après polymérisation doivent être préservées.

D'autre part, le ciment injecté même en petites quantités doit être visualisé en permanence durant l'opération, ce qui n'est pas le cas avec les compositions actuellement commercialisées. Bien que certaines contiennent un radio-opacifiant, une visualisation correcte lors d'une injection percutanée n'est pas obtenue. Ceci amène les praticiens a modifier eux-mêmes les compositions, au risque de modifier du même coup les caractéristiques physiques du ciment, notamment sa viscosité et sa vitesse de durcissement, ainsi que ses propriétés de résistance après polymérisation.

Un autre inconvénient sérieux réside dans le fait que la réaction de polymérisation est exothermique, la température pouvant dépasser 100°C dans le corps vertébral. Cette température excessive entraîne une nécrose osseuse des tissus avoisinants.

Ainsi, les ciments actuellement connus, s'ils sont performants pour la fixation d'implants au squelette, n'ont pas été conçus pour un traitement de stabilisation de corps vertébral par voie percutanée et ne prennent pas en compte les spécificités liées à cette technique.

L'objet de la présente invention est de fournir un matériau adapté à un usage en chirurgie percutanée, en particulier pour le comblement d'un corps vertébral, ledit matériau ayant un comportement intra-opératoire et post-opératoire convenable, à savoir :

-   Fluidité,

- Temps de prise supérieur à 15 minutes,
- Opacité en fluoroscopie,
- Résistance à la compression: au moins 70 Mpa,
- Résistance à la flexion : au moins 50 Mpa,
- Module de flexion: au moins 1 800 Mpa.

Bien entendu, un tel ciment doit être compatible avec un usage médical du point de vue de sa toxicité et de sa biocompatibilité.

Il a été trouvé qu'il était possible de formuler un ciment à base de polyméthylméthacrylate et de méthylméthacrylate monomère répondant au cahier des charges ci-dessus, en incorporant audit ciment des quantités élevées d'une composition radio-opaque comprenant un composé radio-opaque et du phosphate de calcium, ceci sans que les qualités exigées pour l'usage prévu soient altérées.

L'emploi de phosphate de calcium, par exemple d'hydroxyapatite, est connu depuis de nombreuses années dans le domaine de l'orthopédie et de la chirurgie maxillo-faciale. Il est utilisé dans ce cas sous forme de blocs solides ou de revêtement.

Dans le ciment de la présente invention, le phosphate de calcium est utilisé, en combinaison avec le composé radio-opaque, comme renfort d'un composite à base de polymère. Un tel ciment présente l'avantage d'apporter l'effet ostéoconducteur du phosphate de calcium associé à la facilité d'injection caractéristique des ciments acryliques classiques en vertébroplastie. En effet, les particules de phosphate de calcium présentes à la surface du ciment sont reconnues par l'os et peuvent induire l'adhésion, mais aussi une réponse des ostéoblastes. Elle a été étudiée à ce titre, sans que ses propriétés mécaniques soient d'ailleurs mises à profit. La croissance osseuse ainsi stimulée permet de réduire les risques de réaction inflammatoire et augmente la biocompatibilité du ciment. A long terme, la stabilité du corps vertébral est améliorée.

De manière inattendue, il a été trouvé que le phosphate de calcium remplit une autre fonction particulièrement intéressante dans le ciment selon l'invention. En effet, il a été observé que la présence de phosphate de calcium provoque une diminution de la température durant la polymérisation du ciment. Cet effet est particulièrement important lorsque les particules de phosphate de calcium ont un diamètre moyen inférieur à 30$\mu$m. Sans entrer dans les études théoriques, on suppose que les particules de phosphate de calcium agissent comme des dissipateurs de chaleur du fait de l'augmentation de la surface spécifique en contact direct avec l'os. Le résultat de cette diminution de la température de polymérisation, qui peut être de l'ordre de 10°C environ, est une réduction de la nécrose osseuse à l'interface avec le ciment.

Plus précisément, la présente invention a pour objet un ciment fluide à usage médical pour le comblement de corps vertébral, comprenant :

a) 70% à 85 % en poids d'un polymère comprenant un polyméthylméthacrylate et un méthylméthacrylate monomère, et

b) de 15 % à 30 % en poids d'une composition radio-opaque comprenant un composé radio-opaque et du phosphate de calcium.

De préférence, le ciment selon l'invention comprend :

a) au moins 70 % en poids d'un polymère comprenant un polyméthylméthacrylate et un méthylméthacrylate monomère, et

b) au moins 25 % en poids d'une composition radio-opaque comprenant un composé radio-opaque et du phosphate de calcium.

Le ciment selon l'invention présente une bonne fluidité dans les minutes suivant la mise en présence des ingrédients le composant et peut être travaillé jusqu'à 10 minutes et plus après sa préparation. La réaction de polymérisation provoque la prise en masse du polyméthylméthacrylate et du monomère qui disparaissent pour former un ciment polymère solide. La température dans le corps vertébral durant la polymérisation est inférieure à 80°C. Dans la présente demande, le terme "ciment" ou "ciment fluide" correspond au ciment tel qu'il se présente après mélange des ingrédients. La composition du ciment sera considérée comme celle du ciment fluide prêt à l'emploi, avant solidification.

Le polyméthylméthacrylate (ou PMMA) et le méthylméthacrylate monomère (ou MMA) entrant dans la composition du ciment selon l'invention sont ceux utilisés communément pour la préparation de ciments acryliques. Les poudres de PMMA se présentent sous la forme de billes de polymères. La masse molaire de ces poudres est comprise entre 150 000 et 1 500 000 g/mole. Le diamètre moyen des particules est compris entre 30 $\mu$m et 100$\mu$m. Le monomère

est l'ester méthylique de l'acide méthacrylique. Le MMA et le PMMA à usage médical sont disponibles dans le commerce.

La composition radio-opaque représente une fraction importante du ciment. Elle a pour but de permettre d'injecter le ciment sous contrôle continu par fluoroscopie, notamment lors de procédures de vertébroplastie. Elle peut être constituée d'un composé radio-opaque pur ou en mélange avec d'autres ingrédients. Il a été trouvé que la combinaison d'un phosphate de calcium et d'un composé radio-opaque permettait d'obtenir un ciment bien visible durant sa mise en place et bien toléré ensuite par l'organisme. De manière avantageuse, la composition radio-opaque présente dans le ciment selon l'invention comprend un composé radio-opaque et du phosphate de calcium.

Les proportions d'emploi recommandées du composé radio-opaque et du phosphate de calcium, en poids rapporté au poids total de ciment, sont telles que ladite composition radio-opaque comprend 20% à 27% d'un composé radio-opaque et 3% à 6% de phosphate de calcium. De préférence, la composition radio-opaque comprend environ 27% d'un composé radio-opaque et environ 3% de phosphate de calcium.

Le composé radio-opaque peut être choisi parmi les composés connus et compatibles avec un usage médical. Il est de préférence choisi dans le groupe composé du sulfate de barium et du dioxyde de zirconium. Le sulfate de barium ($BaSO_4$) est un radio-opacifiant communément utilisé dans les ciments pour la fixation d'implant, dont l'innocuité est reconnue. Il se présente en général sous forme poudre dont les particules ont un diamètre moyen de 1 à 10 $\mu m$. Le dioxyde de zirconium ($ZrO_2$) peut être utilisé alternativement. Il est introduit sous forme de poudre dont les particules ont un diamètre moyen de 20$\mu m$.

La composition radio-opaque comprend en mélange avec le radio-opacifiant, du phosphate de calcium. Celui-ci est choisi par l'homme du métier parmi les phosphates de calcium de qualité médicale, tels que ceux disponibles dans le commerce. De manière avantageuse, le phosphate de calcium est l'hydroxyde d'apatite. On peut par exemple utiliser une hydroxyapatite phosphocalcique de formule $Ca_{10}(PO_4)_6(OH)_2$, ayant un rapport Ca/P de 1,667 et une granulométrie inférieure à 30$\mu m$. La présence de phosphate de calcium dans la composition radio-opaque apporte un effet triplement bénéfique, d'une part en améliorant l'homogénéité du ciment et par suite sa malléabilité, d'autre part en augmentant sa biocompatibilité et enfin en réduisant la température de polymérisation du ciment.

L'introduction dans le ciment d'une quantité importante d'une composition opacifiante a des répercussions sur ses caractéristiques physiques et chimiques, notamment sa fluidité, sa vitesse de solidification, ainsi que sa résistance mécanique. Pour obtenir des propriétés fonctionnelles optimales, il est recommandé de respecter des proportions d'ingrédients telles que définies par la présente demande, et qui sont également objets de la présente invention.

En particulier, dans le ciment selon l'invention, le polyméthylméthacrylate et le méthylméthacrylate monomère sont avantageusement apportés dans un rapport pondéral compris entre 1 et 2. De préférence le rapport PMMA / MMA est compris entre 1,4 et 1,5. Ainsi, de manière surprenante, il a été déterminé que la formulation optimale est celle où la proportion de PMMA est moindre que dans les formulations classiques, l'ajout de la composition opacifiante se faisant essentiellement au détriment du polyméthylméthacrylate.

Le ciment selon l'invention peut enfin contenir un certain nombre de réactifs favorisant le contrôle de la polymérisation. Notamment, il peut comprendre, outre les ingrédients mentionnés ci-dessus, une quantité efficace d'un ou plusieurs des réactifs suivants: un activateur chimique de polymérisation, un initiateur de polymérisation, un stabilisant. L'homme du métier connaît ces réactifs et maîtrise leur mise en oeuvre.

Un initiateur de réaction peut avantageusement être choisi parmi les catalyseurs de polymérisation tels que le péroxyde de benzoyle (BPO). L'activateur ou accélérateur de la réaction de polymérisation est de préférence la N,N-diméthyl-para-toluidine (DMPT). Le stabilisant, de préférence l'hydroquinone, peut être ajouté pour éviter la polymérisation prématurée du monomère du fait de l'exposition à la chaleur ou à la lumière. Ces réactifs, sont efficaces à des concentrations très faibles, que l'homme de l'art sait ajuster en fonction de la cinétique souhaitée. Ils entrent dans la composition du ciment en des quantités de l'ordre de 0,2% à 2% pour le péroxyde de benzoyle, de 1,5% à 2,5% pour la DMPT, et d'environ 20 ppm pour ce qui concerne l'hydroquinone. Selon une formulation préférée, on emploi 0,4% à 0,6% de péroxyde de benzoyle; 2,4% de DMPT et 20 ppm d'hydroquinone.

Le ciment selon l'invention, une fois prêt, va réagir pour former une masse solide dans un laps de temps relativement court (de quelques minutes à quelques dizaines de minutes), la formulation ici revendiquée faisant prise au plus tôt en 15 minutes. Il est évident que les ingrédients réagissant entre eux doivent être mélangés uniquement au moment de l'emploi. C'est pourquoi il est commode de disposer de deux prémélanges d'ingrédients qu'il suffit de réunir pour préparer le ciment selon l'invention. Ces prémélanges, l'un sous forme de poudre, l'autre sous forme liquide constituent les deux composants d'une composition binaire destinée à la préparation d'un ciment osseux selon l'invention.

Selon l'invention, ladite composition binaire est composée d'un composant liquide L comprenant un méthylméthacrylate monomère et d'un composant en poudre P comprenant un polyméthylméthacrylate, dans laquelle le composant en poudre P comprend de 25% à 50% en poids rapporté au poids de poudre d'une composition radio-opaque.

De manière avantageuse, dans la composition binaire selon l'invention, le composant en poudre P comprend au moins 35 %, de préférence au moins 45 % d'une composition radio-opaque, en poids rapporté au poids de poudre.

Selon une caractéristique intéressante de l'invention, ladite composition radio-opaque comprend un composé radio-opaque et du phosphate de calcium. De manière avantageuse, la composition radio-opaque comprend 30% à 45% d'un composé radio-opaque et 5% à 10% de phosphate de calcium, en poids rapporté au poids de poudre. Selon une formulation préférée, la composition radio-opaque comprend environ 45% d'un composé radio-opaque et environ 5% de phosphate de calcium, en poids rapporté au poids de poudre.

Ledit composé radio-opaque peut être choisi dans le groupe composé du sulfate de barium et du dioxyde de zirconium. En ce qui concerne le phosphate de calcium, on choisit de préférence l'hydroxyde d'apatite.

La composition binaire selon l'invention comprend en outre de préférence une quantité efficace d'un ou plusieurs des réactifs suivants:

- dans le composant liquide L, un activateur chimique de polymérisation;
- dans le composant en poudre P, un initiateur de polymérisation et un stabilisant.

Par exemple, le composant liquide L peut comprendre de 1,5% à 2,5% de DMPT. Le composant en poudre P peut comprendre de 0,2% à 2% de péroxyde de benzoyle et 20 ppm d'hydroquinone. De préférence, le composant liquide L contient 2,4% de DMPT, tandis que le composant en poudre P contient 0,5±0,1% de PBO.

Lors de l'utilisation en bloc opératoire, les deux composants, en poudre et liquide, sont mélangés. A cet instant, la phase poudre se dissout dans la phase liquide, donnant ainsi un mélange qui doit être suffisamment fluide pour pouvoir être injecté dans un corps vertébral. Au cours du mélange, l'activateur et l'initiateur réagissent pour produire des radicaux libres. Ces radicaux initient la réaction de polymérisation conduisant au durcissement progressif du ciment, selon une cinétique souhaitée. Pour une bonne maîtrise de ces critères, la composition binaire selon l'invention est avantageusement formulée de sorte que le composant en poudre P et le composant liquide L sont dans un rapport pondéral P/L compris entre 1 et 2, de préférence entre 1,4 et 1,5.

Pour les praticiens, il est impératif de limiter les manipulations longues et sources d'erreurs au moment d'opérer. C'est pourquoi il est commode de disposer de deux prémélanges d'ingrédients dans des récipients séparés. Un autre objet de la présente invention est donc un dispositif destiné à la préparation d'un ciment fluide à usage médical selon l'invention comprenant :

i) un premier récipient contenant un composant liquide comprenant au moins un monomère de méthylméthacrylate et éventuellement une quantité efficace d'un activateur chimique de polymérisation, de préférence la diméthyl-para-toluidine;

ii) un deuxième récipient contenant un composant en poudre comprenant au moins un polyméthylméthacrylate et une composition radio-opaque telle que décrite précédemment, et

éventuellement une quantité efficace d'un initiateur de polymérisation, de préférence le péroxyde de benzoyle et d'un stabilisant, de préférence l'hydroquinone.

Autrement dit, est revendiqué un dispositif destiné à la préparation d'un ciment fluide à usage médical comprenant un premier et un second récipients, lesdits premier et second récipients contenant respectivement le composant liquide L et le composant en poudre P d'une composition binaire selon l'invention.

Le dispositif selon l'invention peut être avantageusement utilisé pour la préparation d'un ciment fluide à usage médical pour le comblement de corps vertébral.

Encore un objet de la présente invention est un procédé de préparation d'un ciment fluide à usage médical pour le comblement de corps vertébral, comprenant essentiellement l'étape consistant à mélanger en une masse homogène un composant en poudre P et un composant liquide L tels que décrits précédemment.

Selon une variante avantageuse du procédé revendiqué, le composant en poudre P et le composant liquide L sont apportés dans un rapport pondéral P/L compris entre 1 et 2. De préférence, les composants P et L sont apportés dans un rapport pondéral P/L compris entre 1,4 et 1,5.

Le procédé selon l'invention peut être avantageusement mis en oeuvre pour la préparation d'un ciment fluide à usage médical pour le comblement de corps vertébral.

Les exemples suivant permettront de mieux comprendre l'invention, sans toutefois en limiter la portée. Les abréviations suivantes sont utilisées :

PMMA :    polyméthacrylate de méthyle
MMA :     méthacrylate de méthyle
BPO :     péroxyde de benzoyle
BaSO$_4$ :    sulfate de barium
ZrO$_2$ :     dioxyde de zirconium
HAP :     hydroxyapatite phosphocalcique
DMTP :    diméthyl-para-toluidine

HQ : hydroquinone

P/L : rapport phase poudre / phase liquide, en poids.

**EXEMPLE 1 :** Composition binaire 1

| PHASE POUDRE (% en poids) | |
|---|---|
| PMMA | 64,41 |
| BPO | 0,59 |
| $BaSO_4$ | 25,00 |
| HAP | 10,00 |

| PHASE LIQUIDE (% en poids) | |
|---|---|
| MMA | 97,60 |
| DMPT | 2,40 |
| HQ | 0,002 |

avec P/L = 1,41

**EXEMPLE 2 :** Composition binaire 2

| PHASE POUDRE (% en poids) | |
|---|---|
| PMMA | 59,33 |
| BPO | 0,54 |
| $ZrO_2$ | 30,08 |
| HAP | 10,05 |

| PHASE LIQUIDE (% en poids) | |
|---|---|
| MMA | 97,60 |
| DMPT | 2,40 |
| HQ | 0,002 |

avec P/L = 1,57

**EXEMPLE 3** : Composition binaire 3

| PHASE POUDRE (% en poids) | |
|---|---|
| PMMA | 49,43 |
| BPO | 0,45 |
| $ZrO_2$ | 40,01 |
| HAP | 10,11 |

| PHASE LIQUIDE (% en poids) | |
|---|---|
| MMA | 97,60 |
| DMPT | 2,40 |

(suite)

PHASE LIQUIDE (% en poids)
HQ 0,002

avec P/L = 1,47

**EXEMPLE 4** : Composition binaire 4

PHASE POUDRE (% en poids)
PMMA 49,50
BPO 0,45
$ZrO_2$ 45,06
HAP 4,99

PHASE LIQUIDE (% en poids)
MMA 97,60
DMPT 2,40
HQ 0,002

avec P/L = 1,48

**EXEMPLE 5** : Procédé de préparation d'un ciment osseux

• Composant en poudre :

La phase poudre est obtenue par tamisage à 200 $\mu$m des divers ingrédients, puis mélange par brassage pendant une minute, par exemple dans un mélangeur multiflux.

• Composant liquide :

La phase liquide est préparée par dissolution de l'hydroquinone dans le monomère MA.• L'agitation est maintenue jusqu'à dissolution complète. Le DMPT est ensuite ajouté.
Les deux phases sont conditionnées séparément dans des récipients adaptés à leur conservation. Les kits de préparation instantanée comprennent un récipient contenant la phase liquide et un récipient contenant la phase poudre.

• Composition binaire

Lors de l'utilisation en bloc opératoire, les récipients sont ouverts et leur contenu est mélangé. La poudre se dissout rapidement dans la phase liquide donnant un mélange fluide qui est injecté dans le corps vertébral du patient à travers une tubulure adéquate. L'initiateur BPO et l'activateur DMPT réagissent pour former des radicaux libres qui initient la réaction de polymérisation progressive du ciment. Le chirurgien dispose alors d'une quinzaine de minutes pour opérer, en contrôlant la procédure en permanence par fluoroscopie.

**EXEMPLE 6** : Préparation de l'hydroxyapatite

L'hydroxyapatite utilisée est obtenue par précipitation en milieu aqueux. Cette méthode repose sur la neutralisation de l'acide orthophosphorique par l'hydroxyde de calcium, telle que décrite par Wallaeys (Wallaeys R., 1952, "Contribution à l'étude des apatite phosphocalciques", Ann. Chim., 7, pp. 808-848) et reprise par Osaka (Osaka A., Miura Y., Takeuchi K., Asada M. et Takahashi K., 1991, "Calcium apatite prepared from calcium hydroxyde and orthophosphoric acid", J. Mater. Sci. Mat. Med., 2, pp. 51-55). La réaction mise enjeu est la suivante :

$$6\ H_3PO_4 + 10\ Ca(OH)_2 \longrightarrow Ca_{10}(PO_4)_6(OH)_2$$

Cette méthode peu employée a l'énorme avantage d'être non polluante car le milieu réactionnel est l'eau déminéralisée. La réaction ne génère aucun composant toxique risquant de gêner la biocompatibilité du ciment osseux.

Le procédé de fabrication se déroule de la manière suivante:

Après calcination à 900°C, l'hydroxyde de calcium est mis en suspension dans de l'eau déminéralisée, à laquelle on ajoute une solution d'acide phosphorique diluée. Après maturation le produit de réaction est filtré, séché à l'étuve, puis broyé et tamisé aux différentes grilles. Puis la poudre ainsi obtenue est calcinée à une température comprise entre 900°C et 1100°C. Un tamisage final permet de récupérer la fraction de granulométrie inférieure à 30μm.

**EXEMPLE 7 :** Essais Norme ISO

La norme ISO 5833, intitulée "Implants chirurgicaux, implants à base de résine acrylique" définit les caractéristiques requises par la réglementation et les tests standards permettant de quantifier ces caractéristiques. Les compositions décrites dans les exemples 1 à 4 précédents ont été testées.
Une première série d'essais concerne les propriétés du ciment lors de sa mise en oeuvre, à savoir le temps de prise, la température maximale atteinte par le ciment durant la polymérisation, et le temps de pétrissage. Les résultats obtenus pour les quatre compositions sont présentés dans le tableau 1.
Une deuxième série d'essais concerne les propriétés du ciment installé, c'est-à-dire la résistance à la compression, la résistance à la flexion et le module de flexion. Les résultats obtenus avec les compositions 1, 3 et 4 sont présentés dans le tableau 2.
Tous les modes opératoires sont décrits en détail dans la norme ISO 5833.

TABLEAU 1

|  | temps de prise (mn) | température max (°C) | temps de pétrissage (mn) |
|---|---|---|---|
| Composition 1 | 17,80 | 73,9 | 10,75 |
| Composition 2 | 15,66 | 73,3 | 10,20 |
| Composition 3 | 18,46 | 67,9 | 9,50 |
| Composition 4 | 18,05 | 69,5 | 9,00 |

TABLEAU 2

|  | résistance à la compression (Mpa) | résistance à la flexion (Mpa) | module de flexion (Mpa) |
|---|---|---|---|
| Composition 1 | 84,2 | 58,2 | 3345 |
| Composition 3 | 88,4 | 58,1 | 4043 |
| Composition 4 | 74,6 | 57,6 | 3461 |

Les résultats obtenus montrent que les ciments selon l'invention sont conformes à la réglementation en vigueur et peuvent être utilisés en tant qu'implants chirurgicaux.
On note également que leurs caractéristiques répondent au cahier des charges défini plus haut pour des ciments utilisables en vertébroplastie percutanée (temps de prise supérieur à 15 minutes, opacité en fluoroscopie, avec maintien des caractéristiques de résistance à la compression, résistance à la flexion et module de flexion).

**EXEMPLE 8** : Test de température de polymérisation

L'effet de la présence de phosphate de calcium sur la température dans le corps cérébral durant la polymérisation a été mesurée en laboratoire. L'évolution de la température en cours de polymérisation a été mesurée dans un ciment selon l'exemple 1 et dans un ciment témoin dont l'opacifiant est du sulfate de baryum seul (sans hydroxyapatite).

Le résultat de l'essai est présenté dans le tableau 3.

| | COMPOSITION TEST | COMPOSITION TÉMOIN |
|---|---|---|
| PHASE POUDRE (% en poids) | | |
| PMMA: | 64,41 | 64,41 |
| BPO: | 0,59 | 0,59 |
| $BaSO_4$: | 25,00 | 35,00 |
| HAP: | 10,00 | 0 |
| PHASE LIQUIDE (% en poids) | | |
| MMA: | 97,60 | 97,60 |
| DMPT: | 2,40 | 2,40 |
| HQ: | 0,002 | 0,002 |

TABLEAU 3

| | temps de prise (mn) | température maximale (°C) |
|---|---|---|
| Composition test | 17,80 | 73,9 |
| Composition témoin | 17,50 | 81,2 |

On constate que la présence d'hydroxyapatite a eu pour effet de diminuer la température maximale régnant dans ciment pendant la réaction de polymérisation de 7,3 °C.

**Revendications**

1.  Ciment fluide à usage médical pour le comblement de corps vertébral *caractérisé en ce qu'*il comprend :

    a) 70% à 85 % en poids d'un polymère comprenant un polyméthylméthacrylate et un méthylméthacrylate monomère, et
    b) de 15 % à 30 % en poids d'une composition radio-opaque comprenant un composé radio-opaque et du phosphate de calcium.

2.  Ciment selon la revendication 1, *caractérisé en ce que* il comprend :

    a) au moins 70 % en poids d'un polymère comprenant un polyméthylméthacrylate et un méthylméthacrylate monomère, et
    b) au moins 25 % en poids d'une composition radio-opaque comprenant un composé radio-opaque et du phosphate de calcium.

3.  Ciment selon la revendication 1 ou 2, *caractérisé en ce que* ladite composition radio-opaque comprend 20% à 27%, de préférence environ 27%, dudit composé radio-opaque et 3% à 6%, de préférence environ 3%, de phosphate de calcium, en poids rapporté au poids de ciment.

4.  Ciment selon la revendication 3, *caractérisé en ce que* ledit composé radio-opaque est choisi dans le groupe composé du sulfate de barium et du dioxyde de zirconium.

5.  Ciment selon la revendication 1 ou 2, *caractérisé en ce que* le phosphate de calcium est l'hydroxyde d'apatite.

6.  Ciment selon la revendication 1 ou 2, *caractérisé en ce que* le polyméthylméthacrylate et le méthylméthacrylate monomère sont apportés dans un rapport pondéral compris entre 1 et 2, de préférence entre 1,4 et 1,5.

7.  Ciment selon l'une des revendications 1 à 6, *caractérisé en ce qu'*il comprend en outre une quantité efficace d'un ou plusieurs des réactifs suivants: un activateur chimique de polymérisation, de préférence la diméthyl-para-toluidine, un initiateur de polymérisation, de préférence le péroxyde de benzoyle, un stabilisant, de préférence l'hydroquinone.

**8.** Composition binaire destinée à la préparation d'un ciment osseux selon la revendication 1, composée d'un composant liquide L comprenant un méthylméthacrylate monomère et d'un composant en poudre P comprenant un polyméthylméthacrylate, *caractérisée en ce que* le composant en poudre P comprend de 25% à 50% en poids rapporté au poids de poudre d'une composition radio-opaque comprenant un composé radio-opaque et du phosphate de calcium.

**9.** Composition binaire selon la revendication 8, *caractérisée en ce que* le composant en poudre P comprend au moins 35 %, de préférence au moins 45 % en poids rapporté au poids de poudre de ladite composition radio-opaque.

**10.** Composition binaire selon la revendication 8 ou 9, *caractérisée en ce que* la composition radio-opaque comprend 30% à 45%, de préférence environ 45%, dudit composé radio-opaque et 5% à 10%, de préférence environ 5%, de phosphate de calcium, en poids rapporté au poids de poudre.

**11.** Composition binaire selon la revendication 8 ou 9, *caractérisée en ce que* ledit composé radio-opaque est choisi dans le groupe composé du sulfate de barium et du dioxyde de zirconium.

**12.** Composition binaire selon la revendication 8, *caractérisée en ce que* le phosphate de calcium est l'hydroxyde d'apatite.

**13.** Composition binaire selon l'une des revendications 8 à 12, comprenant en outre une quantité efficace d'un ou plusieurs des réactifs suivants:

   - dans le composant liquide L, un activateur chimique de polymérisation, de préférence la diméthyl-para-toluidine;
   - dans le composant en poudre P, un initiateur de polymérisation, de préférence le péroxyde de benzoyle; un stabilisant, de préférence l'hydroquinone.

**14.** Composition binaire selon l'une des revendications 8 à 13 *caractérisé en ce que* le composant en poudre P et le composant liquide L sont dans un rapport pondéral P/L compris entre 1 et 2, de préférence entre 1,4 et 1,5.

**15.** Dispositif destiné à la préparation d'un ciment fluide à usage médical comprenant un premier et un second récipients, *caractérisé en ce que* lesdits premier et second récipients contiennent respectivement le composant liquide L et le composant en poudre P d'une composition binaire selon l'une des revendications 8 à 14.

**16.** Procédé de préparation d'un ciment fluide à usage médical pour le comblement de corps vertébral, comprenant essentiellement l'étape consistant à mélanger les composants en poudre P et liquide L selon l'une des revendications 8 à 14 en une masse homogène.

**17.** Procédé selon la revendication précédente, dans lequel le composant en poudre P et composant liquide L sont apportés dans un rapport pondéral P/L compris entre 1 et 2, de préférence entre 1,4 et 1,5.

**18.** Ciment fluide à usage médical pour le comblement de corps vertébral préparé à l'aide du dispositif selon la revendication 15.

**19.** Ciment fluide à usage médical pour le comblement d'un corps vertébral obtenu par le procédé selon l'une des revendications 17 ou 18.

**Claims**

**1.** Fluid cement, for medical use, for filling the vertebral body, *characterised in that* it comprises:

   a) 70% to 85% by weight of a polymer comprising a polymethylmethacrylate and a methylmethacrylate monomer, and
   b) from 15% to 30% by weight of a radio-opaque composition comprising a radio-opaque compound and calcium phosphate.

**2.** Cement according to claim 1, *characterised in that* it comprises:

a) at least 70% by weight of a polymer comprising a polymethylmethacrylate and a methylmethacrylate monomer, and

b) at least 25% by weight of a radio-opaque composition comprising a radio-opaque compound and calcium phosphate.

3. Cement according to claim 1 or 2, *characterised in that* said radio-opaque composition comprises 20% to 27%, preferably approximately 27%, of said radio-opaque compound and 3% to 6%, preferably approximately 3%, of calcium phosphate, by weight relative to the weight of cement.

4. Cement according to claim 3, *characterised in that* said radio-opaque compound is chosen within the group composed of barium sulphate and zirconium dioxide.

5. Cement according to claim 1 or 2, *characterised in that* the calcium phosphate is apatite hydroxide.

6. Cement according to claim 1 or 2, *characterised in that* the polymethylmethacrylate and the methylmethacrylate monomer are introduced in a weight ratio between 1 and 2, preferably between 1.4 and 1.5.

7. Cement according to one of claims 1 to 6, *characterised in that* it comprises in addition an effective quantity of one or more of the following reagents: a chemical polymerisation activator, preferably dimethylparatoluidine, a polymerisation initiator, preferably benzoyl peroxide, a stabiliser, preferably hydroquinone.

8. Binary composition intended for preparation of a bone cement according to claim 1, composed of a liquid component L comprising a methylmethacrylate monomer and a powder component P comprising a polymethylmethacrylate, *characterised in that* the powder component P comprises from 25% to 50% by weight relative to the weight of powder of a radio-opaque composition comprising a radio-opaque compound and calcium phosphate.

9. Binary composition according to claim 8, *characterised in that* the powder component P comprises at least 35% , preferably at least 45% by weight relative to the weight of powder of said radio-opaque composition.

10. Binary composition according to claim 8 or 9, *characterised in that* the radio-opaque composition comprises 30% to 45%, preferably approximately 45%, of said radio-opaque compound and 5% to 10%, preferably approximately 5%, of calcium phosphate, by weight relative to the weight of powder.

11. Binary composition according to claim 8 or 9, *characterised in that* said radio-opaque compound is chosen within the group composed of barium sulphate and zirconium dioxide.

12. Binary composition according to claim 8, *characterised in that* the calcium phosphate is apatite hydroxide.

13. Binary composition according to claim 8 to 12, comprising in addition an effective quantity of one or more of the following reagents:

- in the liquid component L, a chemical polymerisation activator, preferably dimethylparatoluidine;
- in the powder component P, a polymerisation initiator, preferably benzoyl peroxide; a stabiliser, preferably hydroquinone.

14. Binary composition according to one of claims 8 to 13, *characterised in that* the powder component P and the liquid component L are in a weight ratio P/L between 1 and 2, preferably between 1.4 and 1.5.

15. Device intended for preparation of a fluid cement for medical use comprising a first and a second receptacle, *characterised in that* said first and second receptacles contain respectively the liquid component L and the powder component P of a binary composition according to one of claims 8 to 14.

16. Method of preparing a fluid cement, for medical use, for filling the vertebral body, comprising essentially the step consisting in mixing the components made of powder P and liquid L according to one of claims 8 to 14 into an homogeneous mass.

17. Method according to the preceding claim, in which the powder component P and the liquid component L are provided in a weight ratio P/L between 1 and 2, preferably between 1.4 and 1.5.

**18.** Fluid cement for medical use for filling the vertebral body prepared by means of the device according to claim 15.

**19.** Fluid cemend for medical use for filling the vertebral body obtained by the method according to one of claims 17 or 18.

**Patentansprüche**

**1.** Fluider Zement zur medizinischen Verwendung für die Wirbelkörperauffüllung,
**dadurch gekennzeichnet,**
**dass** er aufweist:

a) 70 bis 85 Gewichtsprozent eines Polymers, das ein Polymethylmethacrylat und ein Methylmethacrylatmonomer aufweist, und
b) 15 bis 30 Gewichtsprozent einer strahlungsundurchlässigen Zusammensetzung, die einen strahlungsundurchlässigen Bestandteil und ein Calciumphosphat aufweist.

**2.** Zement nach Anspruch 2,
**dadurch gekennzeichnet,**
**dass** er aufweist:

a) zumindest 70 Gewichtsprozent eines Polymers, das ein Polymethylmethacrylat und ein Methylmethacrylatmonomer, und
b) zumindest 25 Gewichtsprozent einer strahlungsundurchlässigen Zusammensetzung, die einen strahlungsundurchlässigen Bestandteil und ein Calciumphosphat aufweist.

**3.** Zement nach Anspruch 1 oder 2,
**dadurch gekennzeichnet,**
**dass** die strahlungsundurchlässige Zusammensetzung 20 bis 27 Gewichtsprozent, bevorzugt etwa 27 Gewichtsprozent, des strahlungsundurchlässigen Bestandteils und 3 bis 6 Gewichtsprozent, bevorzugt etwa 3 Gewichtsprozent, an Calciumphosphat bezogen auf das Gewicht des Zements, aufweist.

**4.** Zement nach Anspruch 3,
**dadurch gekennzeichnet,**
**dass** der strahlungsundurchlässige Bestandteil ausgewählt ist aus der Gruppe bestehend aus Bariumsulfat und Zirkoniumdioxid.

**5.** Zement nach Anspruch 1 oder 2,
**dadurch gekennzeichnet,**
**dass** das Calciumphosphat das Hydroxid von Apatit ist.

**6.** Zement nach Anspruch 1 oder 2,
**dadurch gekennzeichnet,**
**dass** das Polymethylmethacrylat und das Methylmethacrylatmonomer in einem Gewichtsverhältnis verwendet werden, das zwischen 1 und 2, bevorzugt zwischen 1,4 und 1,5, liegt.

**7.** Zement nach einem der Ansprüche 1 bis 6,
**dadurch gekennzeichnet,**
**dass** er zusätzlich eine wirksame Menge von einem oder mehreren der folgenden Reagenzmittel enthält: ein chemisches Aktivierungsmittel zur Polymerisation, bevorzugt das Dimethyl-para-toluidin, einen Polymerisationsinitiator, bevorzugt Benzoylperoxid, ein Stabilisierungsmittel, bevorzugt Hydrochinon.

**8.** Binäre Zusammensetzung, die vorgesehen ist zur Herstellung eines Knochenzements nach Anspruch 1, die aus einem flüssigen Bestandteil L mit einem Methylmethacrylatmonomer und einem pulverförmigen Bestandteil P mit einem Polymethylmethacrylat zusammengesetzt ist,
**dadurch gekennzeichnet,**
**dass** der pulverförmige Bestandteil 25 bis 50 Gewichtsprozent bezogen auf das Gewicht des Pulvers einer strahlungsundurchlässigen Zusammensetzung, die einen strahlungsundurchlässigen Bestandteil und ein Calciumphosphat aufweist, aufweist.

9. Binäre Zusammensetzung nach Anspruch 8,
**dadurch gekennzeichnet,**
**dass** der pulverförmige Bestandteil P zumindest 35 Gewichtsprozent, bevorzugt zumindest 45 Gewichtsprozent, bezogen auf das Gewicht der strahlungsundurchlässigen Zusammensetzung aufweist.

10. Binäre Zusammensetzung nach Anspruch 8 oder 9,
**dadurch gekennzeichnet,**
**dass** die strahlungsundurchlässige Zusammensetzung 30 bis 45 Gewichtsprozent, bevorzugt etwa 45 Gewichtsprozent, des strahlungsundurchlässigen Bestandteils und 5 bis 10 Gewichtsprozent, bevorzugt etwa 5 Gewichtsprozent, an Calciumphosphat, bezogen auf das Gewicht des Pulvers, aufweist.

11. Binäre Zusammensetzung nach Anspruch 8 oder 9,
**dadurch gekennzeichnet,**
**dass** der strahlungsundurchlässige Bestandteil ausgewählt ist aus der Gruppe bestehend aus Bariumsulfat und Zirkoniumdioxid.

12. Binäre Zusammensetzung nach Anspruch 8,
**dadurch gekennzeichnet,**
**dass** Calciumphosphat das Hydroxid von Apatit ist.

13. Binäre Zusammensetzung nach einem der Ansprüche 8 bis 12,
die zusätzlich eine wirksame Menge von einem oder mehreren der folgenden Reagenzmittel aufweist:

- in dem flüssigen Bestandteil L, ein chemisches Aktivierungsmittel, bevorzugt Dimethyl-para-toluidin;
- in dem pulverförmigen Bestandteil P, einen Polymerisationsinitiator,

bevorzugt Benzoylperoxid; ein Stabilisierungsmittel, bevorzugt Hydrochinon.

14. Binäre Zusammensetzung nach einem der Ansprüche 8 bis 13,
**dadurch gekennzeichnet,**
**dass** der pulverförmige Bestandteil P und der flüssige Bestandteil L in einem Gewichtsverhältnis P/L vorliegen, das zwischen 1 und 2, bevorzugt zwischen 1,4 und 1,5, liegt.

15. Vorrichtung bestimmt für die Herstellung eines fluiden Zements zur medizinischen Verwendung mit einem ersten und einem zweiten Behälter,
**dadurch gekennzeichnet,**
**dass** die ersten und die zweiten Behälter jeweils den flüssigen Bestandteil L und den pulverförmigen Bestandteil P einer binären Zusammensetzung nach einem der Ansprüche 8 bis 14 enthalten.

16. Verfahren zur Herstellung eines fluiden Zements zur medizinischen Verwendung für die Wirbelkörperauffüllung, das im Wesentlichen den Schritt aufweist, der darin besteht, den pulverförmigen Bestandteil P und den flüssigen Bestandteil L nach einem der Ansprüche 8 bis 14 zu einer homogenen Masse zu vermischen.

17. Verfahren nach dem vorhergehenden Anspruch,
in dem der pulverförmige Bestandteil P und der flüssige Bestandteil L in einem Gewichtsverhältnis P/L verwendet werden, das zwischen 1 und 2, bevorzugt zwischen 1,4 und 1,5, liegt.

18. Fluider Zement zur medizinischen Verwendung für die Wirbelkörperauffüllung hergestellt mit der Hilfe der Vorrichtung nach Anspruch 15.

19. Fluider Zement zur medizinischen Verwendung für die Wirbelkörperauffüllung erhalten durch das Verfahren nach dem der Ansprüche 17 oder 18.